# EUROPÄISCHE PATENTSCHRIFT

(11) **EP 1 487 383 B1**
(45) Veröffentlichungstag und Bekanntmachung des Hinweises auf die Patenterteilung: **13.06.2007**
(21) Anmeldenummer: 03722387.2
(22) Anmeldetag: 02.04.2003
(51) Int. Cl.: A61F 2/44

(54) **WIRBELSÄULENIMPLANTAT AUS KNOCHENMATERIAL**
VERTEBRAL COLUMN IMPLANT CONSISTING OF BONE MATERIAL
IMPLANT RACHIDIEN EN MATIERE OSSEUSE

(30) Priorität: 06.05.2002 DE 10220139
(43) Veröffentlichungstag der Anmeldung: 22.12.2004
(73) Patentinhaber: Tutogen Medical GmbH, 91077 Neunkirchen am Brand (DE)
(72) Erfinder: HENNINGER, Jürgen, 63069 Offenbach (DE); LANDIS, Klaus, 90562 Heroldsberg (DE)
(74) Vertreter: Manitz, Finsterwald & Partner GbR
(86) Internationale Anmeldenummer: PCT/EP2003/003452
(87) Internationale Veröffentlichungsnummer: WO 2003/092559

(56) Entgegenhaltungen:
- WO-A-00/19941
- US-A- 5 989 289
- US-A1- 2002 026 242

## Beschreibung

Die vorliegende Erfindung betrifft ein Wirbelsäulenimplantat zur interkorporellen Fusion nach dem Oberbegriff des Anspruchs 1 (vgl. US2002/026242 A1).

Ein Implantat zur Verbindung von Knochen und insbesondere ein Wirbelsäulenimplantat zur interkorporellen Fusion von Wirbelknochen, das zwischen zwei zu fusionierende Wirbelknochen eingesetzt wird, ist auch aus der DE-A-199 52 939 bekannt.

In der WO 00 19941 A wird ein Verfahren zur Herstellung eines Materials für Implantate beschrieben.

Die Aufgabe der vorliegenden Erfindung besteht darin, ein Implantat zur Fusion von Knochen zu schaffen, das eine verbesserte Kraftverteilung zwischen zwei zu fusionierenden Wirbelknochen gewährleistet, wobei eine besonders gute Anpassung an den Zwischenwirbelraum zwischen benachbarten Halswirbeln erfolgen soll.

Die Lösung dieser Aufgabe erfolgt durch die Merkmale des Anspruchs 1.

Erfindungsgemäß schließen die Außenflächen der beiden Schenkel miteinander einen Winkel von etwa 40 bis 70° ein. Hierdurch ist automatisch eine korrekte Positionierung gegeben.

Ein besonderer Vorteil des Wirbelsäulenimplantats gemäß der Erfindung ist durch das verwendete Material gegeben, das aufgrund seines biologischen Ursprungs und seiner Konservierung keinen Fremdkörper darstellt. Darüber hinaus ergibt sich durch A-förmige Gestaltung mit zwei Schenkeln der Vorteil, dass das Implantat der Form der Kanten von Wirbelkörpern, insbesondere von Halswirbelkörpern, angepasst ist und somit beim Einschieben in den Zwischenwirbelraum automatisch in die richtige Position kommt. Durch die spezielle erfindungsgemäße Form ist das Implantat besonders gut an die natürliche Form der Wirbelkörperendplatten angepasst und bietet so aufgrund der A-Form die größtmögliche Kontaktfläche zu den Endplatten. Dadurch erfolgt eine mehr physiologische Verteilung der Kräfte wodurch Druckspitzen und ein durch diese bedingtes Einsinken des Implantates in die Wirbelkörper vermieden werden.

Besonders vorteilhaft ist die A-Form zudem, da hierdurch sowohl zwischen den Enden der Schenkel wie auch in der zentralen Öffnung lose eingebrachtes Knochenpulver in den Zwischenwirbelraum mitgenommen werden kann. Auf diese Weise kann beim Einschieben des Implantats in den Zwischenwirbelraum automatisch Knochenpulver oder spongiöser Knochen oder dergleichen in den Bereich des Implantats eingebracht werden.

In der Beschreibung, der Zeichnung und den Unteransprüchen sind weitere vorteilhafte Ausgestaltungen des Wirbelsäulenimplantats gemäß der vorliegenden Erfindung angegeben.

Bei einer ersten vorteilhaften Ausgestaltung des erfindungsgemäßen Wirbelsäulenimplantats weist der Körper ausschließlich abgerundete Außenkanten auf. Eine solche Gestaltung des Körpers erleichtert die Applikation des Wirbelsäulenimplantats zwischen den zu fusionierenden Wirbelkörpern, indem die Abrundungen ein Verkanten des Implantats während der Applikation verhindern.

Nach einer weiteren vorteilhaften Ausführungsform der Erfindung weisen die Schenkel des Körpers eine konvex gekrümmte Außenkontur auf. Durch eine solche Gestaltung der äußeren Flächen erfolgt eine besonders gute Anpassung an Wirbekörperendplatten. Auch können die Enden der Schenkel abgerundet, insbesondere etwa halbkreisförmig abgerundet sein, was das Einsetzen des Implantats sowie dessen leichte und korrekte Positionierung begünstigt.

Nach einer weiteren Ausbildung der Erfindung weist die durch die Schenkel und den Quersteg gebildete Öffnung eine abgerundete, insbesondere eine etwa kreisförmige Umfangskontur auf. Eine derart geformte Mittelöffnung lässt sich einerseits besonders einfach herstellen, andererseits ist hierdurch das Implantat mit einer besonders hohen Stabilität versehen.

Nach einer weiteren Ausbildung der Erfindung kann sich die durch die Schenkel und den Quersteg gebildete Mittelöffnung nicht durchgehend von der Vorderseite zur Rückseite des Körpers erstrecken.

Beispielsweise kann diese Öffnung mit einem Boden oder mit einem Zwischenboden versehen sein, wodurch einerseits die Stabilität des Körpers erhöht und andererseits ein Aufnahmeraum für Knochenpulver gebildet werden kann.

Nach einer weiteren Ausbildung der Erfindung kann die Außenkontur des Körpers im Bereich des Querstegs und der sich daran anschließenden freien Enden der Schenkel einen zentralen konkaven Abschnitt aufweisen. Dieser Abschnitt kann dazu dienen, ebenfalls beim Einbringen des Implantats in den Zwischenwirbelraum Knochenpulver oder Knochenspäne einzubringen.

Sofern das Wirbelsäulenimplantat, wie nach einer weiteren vorteilhaften Ausführungsform, einen trapezförmigen Querschnitt aufweist, ist hierdurch auch die Wiederherstellung der physiologischen Krümmung der Wirbelsäule möglich.

Bevorzugt ist das erfindungsgemäße Wirbelsäulenimplantat im Wesentlichen symmetrisch ausgebildet. Es ist jedoch auch eine asymmetrische Formgebung möglich, bei der sich die Gestaltung der beiden Schenkel voneinander unterscheidet.

Nach einer weiteren vorteilhaften Ausführungsform kann der Körper aus kompaktem Knochenmaterial bestehen, wobei sich in der durch die Schenkel und den Quersteg gebildeten Öffnung spongiöses Knochenmaterial befindet. Durch einen derartigen "Innenkern" aus Spongiosa kann die Fusion der benachbarten Wirbelknochen erleichtert werden.

Nach einer weiteren Ausbildung der vorliegenden Erfindung können an dem Körper Aufnahmeöffnungen auf einer oder mehreren Seiten des Körpers vorgesehen sein. In diese Bohrungen können dann Applikationswerkzeuge eingeführt werden, um das Implantat positionsgenau zwischen die zu fusionierenden Wirbel einzubringen.

Der Körper kann erfindungsgemäß aus prozessiertem, konserviertem und sterilem Knochenmaterial humanen Ursprungs, so genanntem Allograft, oder aus prozessiertem, konserviertem und sterilem Knochenmaterial tierischen Ursprungs, so genanntem Xenograft bestehen. Der Körper kann aus massivem kortikalem oder auch aus massivem spongiösem Knochenmaterial gefertigt werden, beispielsweise aus Humerus, Femur, Tibia, oder aus anderen Knochen sowohl von verstorbenen Menschen als auch von Tieren, insbesondere aus Knochenmaterial vom Rind, hergestellt werden.

Als Material wird für das Wirbelsäulenimplantat gemäß der vorliegenden Erfindung ein geeignetes allogenes oder xenogenes Knochenmaterial derart prozessiert, dass es konserviert, lagerfähig sowie steril ist und bestimmungsgemäß eingesetzt werden kann. Die Konservierung des Knochenmaterials kann beispielsweise mittels Gefriertrocknung erfolgen. Ein anderes bevorzugtes Verfahren der Herstellung des Knochenmaterials ist die Prozessierung durch vorzugsweise Lösungsmitteldehydratisierung von nativem Knochenmaterial mittels eines organischen, mit Wasser mischbaren Lösungsmittels, z.B. Methanol, Ethanol, Propanol, Isopropanol, Aceton, Methyl-Ethylketon oder Gemischen dieser Lösungsmittel. Die Konservierung und Sterilisation des Knochenmaterials nach diesem Verfahren ist auch Gegenstand des Patents DE 29 06 650.

Dieses Verfahren dient der Herstellung von Transplantatkonserven und ermöglicht eine Dehydratisierung und Freilegung bis in den Feinbau der Fibrille des Knochenmaterials, so dass das prozessierte Knochenmaterial im histologischen Bild eine dem natürlichen Knochen sehr ähnliche morphologische Struktur aufweist und somit die gewünschten Eigenschaften des Knochenmaterials erhalten bleiben. Dieses Verfahren der Lösungsmitteldehydratisierung hat außerdem den Vorteil, dass im Vergleich zur Gefriertrocknung ein wesentlich geringerer apparativer Aufwand erforderlich ist.

Ferner kann das Knochenmaterial auch durch Lösungsmitteldehydratisierung von nativem Knochen mit anschließender terminaler Sterilisation, insbesondere durch Bestrahlung mit Gamma-Strahlen erzeugt werden. Alternativ kann das spongiöse Knochenmaterial durch aseptische Prozessierung von Knochenmaterial ohne terminale Sterilisation erzeugt werden. Das Ausgangsmaterial des erfindungsgemäßen Knochenimplantats ist menschlicher oder tierischer Knochen von ausreichender Größe.

Zur Entfernung der Antigenität wird der Knochen einer osmotischen Behandlung unterzogen. Weiterhin wird zur Denaturierung löslicher Proteine eine oxidative Behandlung durchgeführt. Zur Optimierung der Virusinaktivierung kann eine ph-Absenkung auf pH 3 erfolgen oder eine Behandlung mit Natronlauge oder einer anderen DNA/RNA zerstörenden Substanz. Der Wasserentzug erfolgt durch organische Lösungsmittel, vorzugsweise Aceton. Die abschließende Sterilisation erfolgt durch energiereiche Strahlung, vorzugsweise γ-Strahlen mit einem Dosismaximum von 25 kGy.

Ein so behandelter Knochen behält seinen natürlichen Mineral-Kollagen-Verbund und dessen Eigenschaften. Darüber hinaus ist ein so behandelter Knochen umbaufähig.

Das erfindungsgemäße Wirbelsäulenimplantat kann insbesondere durch ein Verfahren vorteilhaft hergestellt werden, bei dem menschlicher oder tierischer Tibiaknochen quer zur Knochenlängsachse in zumindest eine Scheibe gesägt wird, wobei aus dieser Scheibe anschließend zwei Wirbelsäulenimplantatkörper gearbeitet werden. Dieses Verfahren beruht auf der Erkenntnis, dass die Außenkontur des Tibiaknochens derjenigen der erfindungsgemäßen Wirbelsäulenimplantatkörper sehr ähnlich ist, so dass sich die Form des erfindungsgemäßen Implantatkörpers vorteilhaft in den Tibiaknochen einbeschreiben lässt.

Nachfolgend wird die vorliegende Erfindung rein exemplarisch anhand von Ausführungsbeispielen eines erfindungsgemäßen Wirbelsäulenimplantats unter Bezugnahme auf die beigefügten Zeichnungen beschrieben. Es zeigen:
- Fig. 1: eine perspektivische Ansicht einer Ausführungsform eines Wirbelsäulenimplantats gemäß der Erfindung;
- Fig. 2: eine vergrößerte Darstellung der Oberflächentextur des Implantats von Fig. 1;
- Fig. 3: eine Draufsicht auf eine weitere Ausführungsform eines Wirbelsäulenimplantats gemäß der Erfindung;
- Fig. 4: mögliche Querschnittsformen des erfindungsgemäßen Wirbelsäulenimplantats;
- Fig. 5: einen Querschnitt durch einen Tibiaknochen mit einbeschriebenen Konturen des erfindungsgemäßen Wirbelsäulenimplantats; und
- Fig. 6: eine Draufsicht auf Ausführungsformen eines Wirbelsäulenimplantats gemäß der vorliegenden Erfindung mit möglichen Bohrungen für die Aufnahme eines Applikationswerkzeugs.

In den Figuren bezeichnen die gleichen Bezugszeichen jeweils die gleichen Komponenten der dargestellten Ausführungsformen.

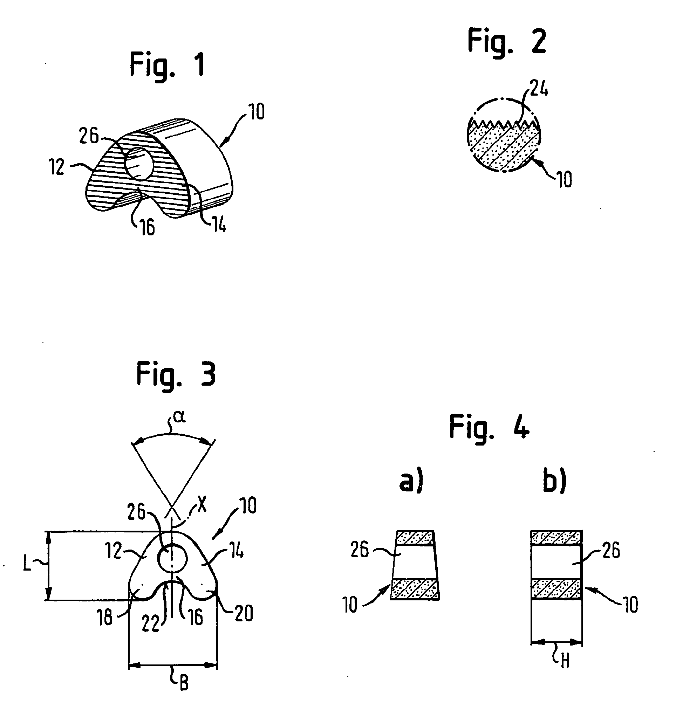

Das in Fig. 1 dargestellte Ausführungsbeispiel eines erfindungsgemäßen Wirbelsäulenimplantats umfasst einen Körper 10, der beispielsweise aus kortikalem, diaphysärem Knochenmaterial z.B. humanen Ursprungs besteht.

Der in Fig. 1 dargestellte Körper 10 weist zwei unter einem spitzen Winkel α (Fig. 3) verlaufende Schenkel 12 und 14 auf, wobei zwischen den Schenkeln 12 und 14 ein die Schenkel verbindender Quersteg 16 vorgesehen ist, so dass der Körper 10 in Draufsicht im Wesentlichen die Form eines A aufweist.

Wie Fig. 1 und insbesondere Fig. 3 zeigt, weist die Außenkontur des "A" ausschließlich abgerundete Kanten auf. Außerdem weisen die Schenkel 12 und 14 eine leicht konvex gekrümmte Außenkontur auf. Wie ferner zu erkennen ist, ist die Breite der Schenkel 12 und 14 über der Längserstreckung nicht einheitlich. Vielmehr sind die beiden Schenkel 12 und 14 an ihren äußeren freien Enden relativ breit. Im Bereich des Scheitels des A liegt demgegenüber eine geringere Breite der Schenkel vor.

Die freien Enden 18, 20 der Schenkel 12 und 14 (Fig. 3) sind etwa halbkreisförmig abgerundet. Die Außenkontur des Körpers 10 im Bereich des Querstegs 16 und der sich daran anschließenden freien Enden 18, 20 der Schenkel 12, 14 weist einen konkaven Abschnitt 22 auf, der stetig in die ansonsten vollständig konvex gekrümmte Umfangskontur des Körpers 10 übergeht.

Wie die Fig. 1 und 2 zeigen, kann die dargestellte Oberseite oder auch die Unterseite des Körpers 10 eine Riffelung 14 aufweisen, die eine Dislokation des Implantats im Wirbelzwischenraum verhindert.

Die in den Figuren dargestellten Ausführungsformen des erfindungsgemäßen Wirbelsäulenimplantats sind zu dessen Mittelachse X symmetrisch ausgebildet. Es sei jedoch auch darauf hingewiesen, dass auch unsymmetrische Ausführungsformen denkbar sind, bei denen beispielsweise die Länge oder die Gestaltung der beiden Schenkel 18 und 20 unterschiedlich ist.

Wie die Figuren ferner zeigen, ist durch die beiden Schenkel 12 und 14 sowie den Quersteg 16 eine Öffnung 26 gebildet, die beim dargestellten Ausführungsbeispiel von der Vorderseite zur Rückseite des Körpers 10 durchgehend und mit kreisförmigem Querschnitt ausgebildet ist.

Fig. 3 zeigt eine Draufsicht auf eine Ausführungsform eines Körpers 10 eines Wirbelsäulenimplantats, wobei die Breite B, die Länge L und die Höhe H (vgl. Fig. 4b)) wie folgt gewählt werden können: Bei lumbalen Anwendungen kann die Länge L etwa 30 bis 60 mm, die Breite B etwa 8 bis 20 mm und die Höhe H etwa 6 bis 18 mm betragen. Bei cervikalen Anwendungen kann die Länge L etwa 8 bis 18 mm, die Breite B etwa 8 bis 18 mm und die Höhe H etwa 4 bis 15 mm betragen. Bei dem in Fig. 3 dargestellten Ausführungsbeispiel beträgt die Länge L etwa 11 mm, die Breite B etwa 14 mm und die Höhe H etwa 8 mm.

Der Winkel α, den die Außenflächen der beiden Schenkel 12 und 14 miteinander einschließen, liegt in einem Bereich von etwa 40 bis 70°. Bei dem in Fig. 3 dargestellten Ausführungsbeispiel beträgt der Winkel α etwa 55°.

Fig. 4a) und 4b) zeigen mögliche Querschnittsformen des Körpers 10 entlang der Mittelachse X von Fig. 3. Wie aus Fig. 4a) erkennbar ist, kann der Querschnitt des Körpers 10 trapezförmig oder aber, wie in Fig. 4b) gezeigt ist, im Wesentlichen rechteckig sein. Die in Fig. 4a) dargestellten schräg verlaufenden Seiten können unter einem Winkel von etwa 3 bis 6 ° von der Rechteckform abweisen. An dieser Stelle sei nochmals darauf hingewiesen, dass die Darstellung der Fig. 4 nicht maßstäblich zu denjenigen von Fig. 3 ist.

Grundsätzlich besteht die Möglichkeit, dass das erfindungsgemäße Wirbelsäulenimplantat massiv ausgebildet wird, beispielsweise aus kortikalem oder aus spongiösem Knochen. Eine alternative Ausführungsform der Erfindung sieht vor, dass das Wirbelsäulenimplantat - ebenfalls aus kortikalem oder spongiösem Knochen - als Hohlkörper ausgebildet wird.

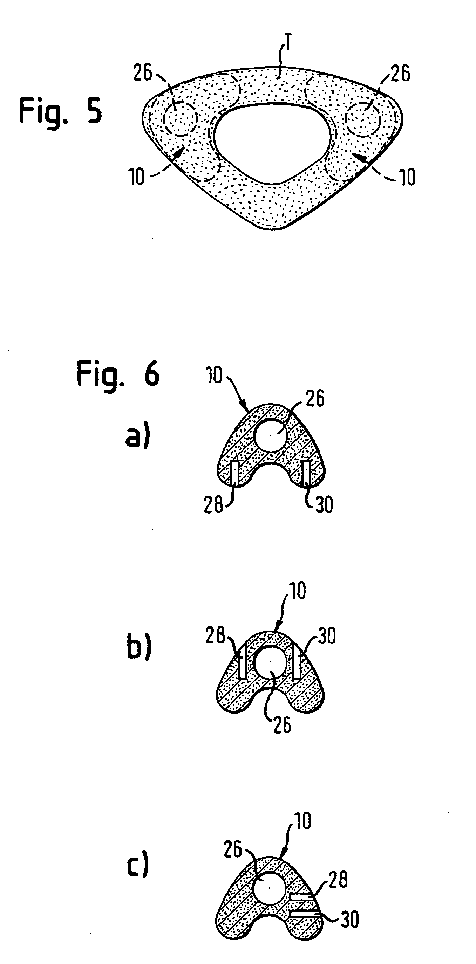

Fig. 5 zeigt im Querschnitt einen Tibiaknochen T, wobei die Außenkonturen zweier Wirbelsäulenimplantate 10 gemäß der Erfindung in den Tibiaquerschnitt einbeschrieben sind. Wie gut zu erkennen ist, eignet sich der Tibiaknochen in besonders guter Weise zur Herstellung des erfindungsgemäßen Wirbelsäulenimplantatkörpers, da die Außenkontur und die Innenkontur des Tibiaknochens T der Außenkontur des Körpers 10 nahezu entspricht. Bei einem besonders vorteilhaften Verfahren zur Herstellung des Körpers 10 wird deshalb menschlicher oder tierischer Tibiaknochen quer zu dessen Längsachse in Scheiben gesägt, wobei anschließend zwei der in den Figuren dargestellten Körper 10 aus der so gewonnenen Knochenscheibe herausgearbeitet werden, beispielsweise durch Fräsen.

Die Fig. 6a) bis c) zeigen verschiedene Ausführungsformen eines Wirbelsäulenimplantats 10 mit Lochbohrungen 28, 30 für die Aufnahme in ein Applikationswerkzeug. Die Lochbohrungen können unterschiedliche Tiefe aufweisen und in verschiedenen Winkeln zueinander stehen. Auch ist es möglich, lediglich eine Lochbohrung vorzusehen. Grundsätzlich sind die Lochbohrungen in derjenigen Ebene vorgesehen, in der das Wirbelsäulenimplantat in den Wirbelzwischenraum eingeführt werden kann. Je nach Applikationsart werden die Lochbohrungen 28, 30 an den freien Enden der Schenkel 12, 14 (Fig. 6a)), im Bereich des spitzen Endes des "A" (Fig. 6b)) oder an einer Seite der Schenkel 12, 14 (Fig. 6c)) positioniert.

### Bezugszeichenliste

- 10: Körper
- 12, 14: Schenkel
- 16: Quersteg
- 18, 20: freie Enden der Schenkel
- 22: zentraler konkaver Abschnitt
- 24: Riffelung
- 26: Öffnung
- 28,30: Lochbohrung

- B: Breite
- H: Höhe
- L: Länge
- T: Tibiaknochen
- X: Mittelachse
- α: Winkel zwischen den Schenkeln

## Patentansprüche

1. Wirbelsäulenimplantat zur interkorporellen Fusion an der Wirbelsäule, bestehend aus einem Körper (10) aus konserviertem Knochenmaterial, der einen im wesentlichen rechteckigen oder trapezförmigen Querschnitt besitzt
**dadurch gekennzeichnet,**
**dass** der Körper (10) zwei unter einem spitzen Winkel (α) verlaufende Schenkel (12, 14) und einen die Schenkel (12, 14) verbindenden Quersteg (16) aufweist, so dass der Körper (10) in Draufsicht im Wesentlichen die Form eines A aufweist und
**dass** die seitlichen Außenflächen der beiden Schenkel (12, 14) einen Winkel von etwa 40 -70° einschließen.

2. Wirbelsäülenimplantat nach Anspruch 1,
**dadurch gekennzeichnet, dass**
die Außenkontur des "A" ausschließlich abgerundete Kanten aufweist.

3. Wirbelsäulenimplantat nach Anspruch 1,
**dadurch gekennzeichnet, dass**
die Schenkel (12, 14) eine konvex gekrümmte Außenkontur aufweisen.

4. Wirbelsäulenimplantat nach Anspruch 1,
**dadurch gekennzeichnet, dass**
die Enden (18, 20) der Schenkel (12, 14) insbesondere etwa halbkreisförmig abgerundet sind.

5. Wirbelsäulenimplantat nach Anspruch 1,
**dadurch gekennzeichnet, dass**
die durch die Schenkel (12, 14) und den Quersteg (16) gebildete Öffnung (26) eine abgerundete, insbesondere eine etwa kreisförmige Umfangskontur aufweist.

6. Wirbelsäulenimplantat nach Anspruch 1,
**dadurch gekennzeichnet, dass**
die durch die Schenkel (12, 14) und den Quersteg (16) gebildete Öffnung (26) sich nicht durchgehend von der Vorderseite zu der Rückseite des Körpers (10) erstreckt.

7. Wirbelsäulenimplantat nach Anspruch 1,
**dadurch gekennzeichnet, dass**
die Außenkontur des Körpers (10) im Bereich des Querstegs (16) und der sich daran anschließenden freien Enden (18, 20) der Schenkel (12, 14) einen zentralen konkaven Abschnitt (22) aufweist.

8. Wirbelsäulenimplantat nach Anspruch 1,
**dadurch gekennzeichnet, dass**
zumindest eine Außenfläche des Körpers (10) eine Riffelung (24) aufweist, die eine Dislokation des Implantats im Wirbelzwischenraum verhindert.

9. Wirbelsäulenimplantat nach Anspruch 1,
**dadurch gekennzeichnet, dass**
der Körper (10) symmetrisch ausgebildet ist.

10. Wirbelsäulenimplantat nach Anspruch 1,
**dadurch gekennzeichnet, dass**
der Körper (10) aus Kompakta besteht und dass sich in der durch die Schenkel (12, 14) und den Quersteg (16) gebildeten Öffnung (26) spongiöses Knochenmaterial befindet.

11. Wirbelsäulenimplantat nach Anspruch 1,
**dadurch gekennzeichnet, dass**
der Körper (10) aus konserviertem und sterilem Knochenmaterial humanen oder tierischen Ursprungs, insbesondere aus konserviertem und sterilem bovinem Knochenmaterial besteht.

12. Verfahren zur Herstellung eines Wirbelsäulenimplantats nach zumindest einem der vorstehenden Ansprüche,
**dadurch gekennzeichnet, dass**
menschlicher oder tierischer Tibiaknochen quer zu dessen Längsachse in zumindest eine Scheibe gesägt wird, und dass anschließend aus dieser Scheibe zwei Körper (10) gearbeitet werden.

## Claims

1. A spinal column implant for interbody fusion to the spinal column, consisting of a body (10) of preserved bone material which has a substantially rectangular or trapezoidal cross-section,
**characterized in that** the body (10) has two limbs (12, 14) extending at an acute angle (α) and a transverse web (16) connecting the limbs (12, 14) so that the body (10) substantially has the shape of an A in plan view; and
**in that** the lateral outer surfaces of the two limbs (12, 14) include an angle from approximately 40 - 70°C.

2. A spinal column implant in accordance with claim 1, **characterized in that** the outer contour of the "A" has only rounded edges.

3. A spinal column implant in accordance with claim 1, **characterized in that** the limbs (12, 14) have a convexly curved outer contour.

4. A spinal column implant in accordance with claim 1, **characterized in that** the ends (18, 20) of the limbs (12, 14) are in particular rounded in approximately semi-circular shape.

5. A spinal column implant in accordance with claim 1, **characterized in that** the opening (26) formed by the limbs (12, 14) and by the transverse web (16) has a rounded peripheral contour, in particular an approximately circular outer contour.

6. A spinal column implant in accordance with claim 1, **characterized in that** the opening (26) formed by the limbs (12, 14) and by the transverse web (16) does not extend in a through-going manner from the front side to the rear side of the body (10).

7. A spinal column implant in accordance with claim 1, **characterized in that** the outer contour of the body (10) has a central concave section (22) in the region of the transverse web (16) and of the free ends (18, 20) of the limbs (12, 14) adjoining it.

8. A spinal column implant in accordance with claim 1, **characterized in that** at least one outer surface of the body (10) has a grooving (24) which prevents a dislocation of the implant in the intervertebral space.

9. A spinal column implant in accordance with claim 1, **characterized in that** the body (10) is symmetrical.

10. A spinal column implant in accordance with claim 1, **characterized in that** the body (10) consists of compacta; and **in that** spongious bone material is located in the opening (26) formed by the limbs (12, 14) and by the transverse web (16).

11. A spinal column implant in accordance with claim 1, **characterized in that** the body (10) consists of preserved and sterile bone material of human or animal origin, in particular of preserved and sterile bovine bone material.

12. A method for the manufacture of a spinal column implant in accordance with at least one of the preceding claims, **characterized in that** human or animal tibia bone is sawn transversely to its longitudinal axis into at least one slice; and **in that** two bodies (10) are subsequently worked from this slice.

## Revendications

1. Implant rachidien pour la fusion intercorporelle au niveau de la colonne vertébrale, comprenant un corps (10) en matériau osseux conservé qui possède une section sensiblement rectangulaire ou trapézoïdale,
**caractérisé en ce que** le corps (10) comporte deux bras (12, 14) qui s'étendent sous un angle aigu (α) et une barrette transversale (16) qui relie les bras (12, 14) de telle façon que le corps (10) présente en vue de dessus essentiellement la forme d'un A, et
**en ce que** les surfaces extérieures latérales des deux bras (12, 14) définissent un angle d'environ 40 à 70°.

2. Implant rachidien selon la revendication 1, **caractérisé en ce que** le contour extérieur du "A" présente exclusivement des arêtes arrondies.

3. Implant rachidien selon la revendication 1, **caractérisé en ce que** les bras (12, 14) présentent un contour extérieur à courbure convexe.

4. Implant rachidien selon la revendication 1, **caractérisé en ce que** les extrémités (18, 20) des bras (12, 14) sont arrondies en particulier sous forme de demi-cercle.

5. Implant rachidien selon la revendication 1, **caractérisé en ce que** l'ouverture (20) formée par les bras (12, 14) et par la barrette transversale (16) présente un contour périphérique arrondi, en particulier de forme approximativement circulaire.

6. Implant rachidien selon la revendication 1, **caractérisé en ce que** l'ouverture (26) formée par les bras (12, 14) et par la barrette transversale (16) ne s'étend pas totalement depuis la face antérieure vers la face postérieure du corps (10).

7. Implant rachidien selon la revendication 1, **caractérisé en ce que** le contour extérieur du corps (10) présente un tronçon central concave (22) dans la région de la barrette transversale (16) et des extrémités libres (18, 20), qui s'y raccordent, des bras (12, 14).

8. Implant rachidien selon la revendication 1, **caractérisé en ce qu'**une surface extérieure du corps (10) au moins présente des cannelures (24) qui empêchent une dislocation de l'implant dans l'espace intervertébral.

9. Implant rachidien selon la revendication 1, **caractérisé en ce que** le corps (10) est réalisé symétrique.

10. Implant rachidien selon la revendication 1, **caractérisé en ce que** le corps (10) est réalisé en matériau compact, et **en ce qu'**un matériau osseux spongieux se trouve dans l'ouverture (26) formée par les bras (12, 14) et par la barrette transversale (16).

11. Implant rachidien selon la revendication 1, **caractérisé en ce que** le corps (10) est réalisé en matériau osseux conservé et stérile d'origine humaine ou animale, en particulier en matériau osseux bovin conservé et stérile.

12. Procédé pour réaliser un implant rachidien selon l'une au moins des revendications précédentes, **caractérisé en ce qu'**un os de tibia humain ou animal est scié transversalement à son axe longitudinal pour former au moins un disque, et **en ce que** l'on usine ensuite deux corps (10) à partir de ce disque.
